# EUROPÄISCHE PATENTSCHRIFT

(11) **EP 3 393 391 B1**
(45) Veröffentlichungstag und Bekanntmachung des Hinweises auf die Patenterteilung: **02.11.2022**
(21) Anmeldenummer: 16829399.1
(22) Anmeldetag: 15.12.2016
(51) Int. Cl.: A61B 90/00, A61B 34/20

(54) **VORRICHTUNG FÜR DIE INTRAOPERATIVE BILDGESTEUERTE NAVIGATION BEI CHIRURGISCHEN EINGRIFFEN IM BEREICH DER WIRBELSÄULE UND IM DARAN ANGRENZENDEN THORAX-, BECKEN- ODER KOPFBEREICH**
DEVICE FOR INTRAOPERATIVE IMAGE-GUIDED NAVIGATION DURING SURGICAL INTERVENTIONS IN THE VICINITY OF THE SPINE AND THE ADJOINING THORACIC, PELVIC OR HEAD AREA
DISPOSITIF POUR LA NAVIGATION GUIDÉE PAR IMAGE INTRA-OPÉRATOIRE LORS D'INTERVENTIONS CHIRURGICALES DANS LA RÉGION DE LA COLONNE VERTÉBRALE ET DANS LA ZONE ADJACENTE DU THORAX, DU BASSIN OU DE LA TÊTE

(30) Priorität: 22.12.2015 EP 15405076
(43) Veröffentlichungstag der Anmeldung: 31.10.2018
(73) Patentinhaber: SpineMind AG, 6370 Stans (CH)
(72) Erfinder: JESZENSZKY, Dezsö János, 8700 Küsnacht (CH); FEKETE, Tamás Fülöp, 8008 Zürich (CH)
(74) Vertreter: EGLI-EUROPEAN PATENT ATTORNEYS
(86) Internationale Anmeldenummer: PCT/CH2016/000155
(87) Internationale Veröffentlichungsnummer: WO 2017/106980

(56) Entgegenhaltungen:
- WO-A2-2013/001031
- DE-A1- 10 015 826
- US-A1- 2009 318 756

## Beschreibung

Die Erfindung betrifft eine Vorrichtung für die intraoperative bildgesteuerte Navigation bei chirurgischen Eingriffen gemäss Anspruch 1.

Aus dem Stand der Technik sind diverse Vorrichtungen und Verfahren für die intraoperative bildgesteuerte Navigation bekannt, die dem Chirurgen während einer Operation die Ausrichtung der verwendeten Instrumente oder Implantate relativ zum Patienten visualisieren. Das grundlegende Ziel dieser Navigationsvorrichtungen und -verfahren ist die Verbesserung der räumlichen Orientierung des Chirurgen während der Operation und damit letztlich die Erhöhung der Sicherheit des Patienten. Eine Vielzahl von Navigationssystemen basieren auf der als Registrierung bezeichneten Korrelierung bzw. Referenzierung von präoperativen Bilddaten, beispielsweise Computertomographie-(CT)- und/oder Magnetresonanztomographie-(MRT)-Bilddaten, mit dem intraoperativen Koordinatensystem des Patienten.

Grundsätzlich sind solche Registrierungsverfahren aus der medizinischen Bildverarbeitung bekannt. Dort dienen sie dazu, zwei oder mehrere Bilder derselben Szene, oder zumindest ähnlicher Szenen, bestmöglich in Übereinstimmung zu bringen, um aus ihrer Kombination bessere Erkenntnisse zu gewinnen. So werden z. B. MRT-Bilder, die Weichteilgewebe oder Gehirnstrukturen gut darstellen, mit CT-Bildern überlagert, um etwa die räumlichen Verhältnisse zwischen ossären Strukturen (CT-Bilder) und neutralen Elementen (MRT-Bilder) nachvollziehen zu können. Die zu registrierenden Bilder unterscheiden sich im Allgemeinen voneinander, etwa weil sie von unterschiedlichen Positionen, zu unterschiedlichen Zeitpunkten oder mit unterschiedlichen Verfahren (Modalitäten) aufgenommen wurden. Für die Registrierung wird in der Regel eines der Bilder als Referenzbild festgelegt, während die übrigen Objektbilder durch die Berechnung einer ausgleichenden Transformation, etwa auf Basis der Minimierung der mittlerem Fehlerquadrate, optimal an das Referenzbild angepasst werden.

In analoger Weise wird für den Registrierungsprozess bei der bildgesteuerten Navigation eine Transformation zwischen dem Koordinatensystem des Patienten und dem präoperativen Bilddatensatz durchgeführt, die auf der Identifizierung von Merkmalen bzw. Markern beruht, die sich sowohl in den präoperativen Bilddaten als auch in der realen Operationssituation am Patienten darstellen lassen. Das Ergebnis der Registrierung ist zumeist eine affine Transformation, welche eine Rotation, Translation und Skalierung der Bilddaten auf das Patienten-Koordinatensystem beinhaltet.

Für die Repräsentation des Patienten-Koordinatensystems werden einerseits sogenannte anatomische oder künstliche Landmarken und andererseits intraoperative Bildgebungsverfahren verwendet.

Die Registrierung über anatomische Landmarken erfordert die Auswahl geeigneter anatomischer Punkte am Patienten. In der Wirbelsäulenchirurgie werden die anatomischen Landmarken derart gewählt, dass sie etwa auf der Knochenoberfläche des Wirbels liegen. Um diese abzutasten, wird in der Regel die Knochenoberfläche der Wirbel freigelegt. Die gewählten Merkmale sollten das Operationsgebiet möglichst umschliessen, da dadurch die Genauigkeit der Registrierung steigt. Auf anatomischen Landmarken basierende Verfahren sind jedoch häufig mit einem grossen Zeitaufwand verbunden. So ist zwar beim Vorliegen einer normalen Anatomie das genaue Auffinden der Landmarken relativ genau möglich. Im Gegensatz dazu ist die Landmarkenerkennung bei Patienten mit wirbelsäulenpathologischen Veränderungen, etwa bei Tumoren, angeborenen, degenerativen oder traumatischen Veränderungen, deutlich erschwert und weniger genau. Oft resultiert dies in einer nur unbefriedigenden Navigationsgenauigkeit. Zudem ist dieses Verfahren sehr zeitaufwendig.

Eine Weiterentwicklung stellt die Verwendung künstlicher Landmarken und intraoperativer Bildgebungsverfahren dar, die die künstlichen Landmarken mitabbilden. Systeme, die auf künstlichen Landmarken basieren, bestehen in der Regel aus einem Lagesensor, einem Computer, einem Anzeigegerät und verschiedenen als künstliche Landmarken dienende Lokalisatoren. Die Lokalisatoren sind geeignete Referenzkörper, deren räumliche Lage durch den verwendeten Lagesensor gemessen wird. Für die Navigation werden diese Lokalisatoren z.B. in Form von Knochenschrauben am Patienten oder an Implantaten befestigt. Die Positionen künstlicher Landmarken können im Vergleich zu anatomischen Landmarken wesentlich exakter in den Bilddaten und in der Realität, d.h. in der realen Operationssituation am Patienten, vermessen werden, wodurch die Registriergenauigkeit gesteigert wird. Allerdings unterliegen auch künstliche Landmarken Störeinflüssen. Dies sind vornehmlich intraoperative Verschiebungen der Landmarken, bedingt durch intraoperative Manipulationen am Patienten oder auch versehentliches Verschieben während der Operation, was wiederum sehr zeitaufwändige Mehrfachregistrierungen notwendig macht. Mit der Erkenntnis, dass u.U. erst zu spät bemerkt werden könnte, dass sich künstliche Landmarken während der Operation verschoben haben, sinkt letztlich das grundsätzliche Vertrauen in die Navigation.

Demgegenüber ist beim Einsatz von intraoperativen Bildgebungsverfahren der Zeitaufwand deutlich geringer und die Registrierung des Patienten-Koordinatensystems mit den Bilddaten wesentlich reproduzierbarer. Vor allem die röntgenbasierte Registrierung oder eine Registrierung basierend auf 2D- oder 3D-Bildverstärkern bzw. intraoperativem CT ist hierbei sehr etabliert. Dabei wird beispielsweise ein präoperativ gewonnenes, CT-basiertes zwei- oder dreidimensionales Bild der Wirbelsäule mit einem intraoperativen zweidimensionalen Röntgenbild der Wirbelsäule über Software-Algorithmen fusioniert oder ein 3D-Datensatz wird intraoperativ erstellt (intraoperatives CT oder 3D-Bildverstärker), wodurch es insbesondere möglich ist, dass der Operateur etwa die Position der Operationsinstrumente und die Lage eingebrachter Implantate, etwa von Schrauben, intraoperativ in Echtzeit an einem dreidimensionalen Datensatz der Wirbelsäule verfolgen kann. Zudem ermöglicht die intraoperative Bildgebung mittels Röntgenstrahlen, noch während der Operation die Lage der implantierten Implantate zu kontrollieren und ggf. zu korrigieren, wobei dies durch wiederholte Bildgebung mittels Röntgenstrahlen zu höheren und meistens beträchtlichen Strahlendosen führt. Auch die Beurteilung und Anpassung des Alignements der Wirbelsäule ist mit intraoperativen Bildgebungsverfahren möglich. Jedoch sind viele Geräte für die intraoperative Bildgebung häufig sehr unflexibel und nehmen viel Platz ein, so dass sie auf den unmittelbaren Operationsbereich um den Patienten herum sehr einschränkend wirken. Zudem kommt - wie zuvor bereits erwähnt - bei jenen Bildgebungsverfahren, die auf Röntgenstrahlung basieren, als zusätzlicher Nachteil die Strahlenbelastung für den Patienten und das medizinische Personal hinzu.

Als Alternative hierzu sind aus dem Stand der Technik ferner Navigationsverfahren und Navigationsvorrichtungen bekannt, welche auf einer intraoperativen Ultraschall-Bildgebung basieren, wobei anstelle des oben beschriebenen intraoperativen röntgenbasierten Bilds ein intraoperativ genommener Ultraschall-Datensatz mit präoperativen Bilddaten fusioniert wird, um vorzugsweise in Echtzeit einen dreidimensionalen Datensatz der Wirbelsäule zu erhalten. Eine derartige Vorrichtung ist beispielsweise aus der internationalen Patentanmeldung WO 96/11624 bekannt. Dabei werden die Ultraschall-Datensätze von ausserhalb des Körpers in Richtung des Körperinneren aufgenommen, indem mit dem Ultraschallkopf jene Rückenpartie, die unmittelbar an den zu operierenden Knochenbereich angrenzt, von aussen, d.h. zum Beispiel auf der Rückenhaut aufsitzend, abgefahren wird.

Die Patentanmeldung WO 2013/001031 offenbart ein Verfahren und eine Vorrichtung zum Darstellen eines Objektes, insbesondere eines biologischen Gewebes, wobei das Verfahren die Schritte aufweist: a) Erzeugen eines ersten Bildes zumindest eines Teilbereichs des Objekts mit Hilfe einer ersten Vorrichtung; b) Erzeugen eines zweiten Bildes zumindest eines Teilbereichs des Objekts mit Hilfe einer zweiten Vorrichtung; c) Ermitteln von ersten Koordinaten zumindest einiger Bildpunkte des zweiten Bildes in einem ersten Koordinatensystem; d) Ermitteln von zweiten Koordinaten der Bildpunkte des zweiten Bildes durch Abbilden der ersten Koordinaten in ein zweites Koordinatensystem, das von dem ersten Koordinatensystem verschieden und der ersten Vorrichtung zugeordnet ist; und e) Erzeugen eines kombinierten Bildes des Objekts aus dem ersten und dem zweiten Bild unter Verwendung der ermittelten zweiten Koordinaten der Bildpunkte des zweiten Bildes.

Bei der klassischen röntgenbasierten wie auch bei der ultraschallbasierten intraoperativen Bildgebung wird das zu operierende Objekt jedoch entweder nur in Projektion oder aber nur ausschnittsweise in Form eines Konturbildes eines Teilbereichs abgebildet. Diese Projektionsbilder bzw. Teilbereichsbilder werden im Weiteren als Referenz genutzt, um mit präoperativen Bilddaten überlagert zu werden, auf deren Basis dann ein auf die intraoperativen Bilddaten referenziertes, virtuelles Echtzeit-Bild generiert wird. Dieses virtuelle Bild gibt jedoch nicht das zu operierende Objekt selbst in Echtzeit wieder, sondern nur dessen Echtzeit-Position. Die Projektionsbilder bzw. die Teilbereichskonturbilder sind allerdings - da sie gerade eben nur die Projektion bzw. nur einen Teilbereich wiedergeben - immanent nicht für die bildliche Wiedergabe des gesamten zu operierenden Objekts in Echtzeit bzw. der aktuellen intraoperativen Situation geeignet.

Aufgabe der vorliegenden Erfindung ist es daher, eine Vorrichtung für die intraoperative, bildgesteuerte Navigation bei chirurgischen Eingriffen an einem zu operierenden Objekt an der Wirbelsäule und/oder im an die Wirbelsäule angrenzenden Thorax-, Becken- oder Kopfbereich anzugeben, die es erlaubt, ein virtuelles, dreidimensionales Echtzeit-Bild des zu operierenden Objekts zu erzeugen, das zum Zwecke der Navigation auf ein stationäres Bezugssystem referenziert ist. Zudem soll die Vorrichtung den Operateur während der Operation nicht behindern und nicht-sensitiv gegen etwaige intraoperativ Störeinwirkungen sein, so dass stets eine hinreichende Navigationsgenauigkeit gewährleistet ist.

Diese Aufgabe wird durch eine Vorrichtung für die intraoperative, bildgesteuerte Navigation nach Anspruch 1 gelöst. Vorteilhafte Ausgestaltungen der Erfindung sind Gegenstand der abhängigen Ansprüche.

Gemäss der Erfindung weist die Vorrichtung mehrere nicht-röntgenbasierte Detektionseinrichtungen, die jeweils dazu ausgebildet sind, verteilt um das zu operierende Objekt im Bereich der Wirbelsäule und/oder im daran angrenzenden Thorax-, Becken- oder Kopfbereich angeordnet zu werden und intraoperativ in Echtzeit Bild- und Lagedaten zu erfassen, die jeweils Informationen über zumindest die Aussenkontur wenigstens eines Teilbereichs des zu operierenden Objekts sowie über den Abstand und die räumliche Orientierung des Teilbereichs relativ zur jeweiligen Detektionseinrichtung umfassen.

Als Aussenkontur des zu operierenden Knochenbereichs wird im Sinne der vorliegenden Erfindung die dreidimensionale Oberfläche des zu operierenden Objekts verstanden. Die Lage des Teilbereichs relativ zur jeweiligen Detektionseinrichtung ist im Sinne der vorliegenden Erfindung als die räumliche Anordnung der erfassten dreidimensionalen Oberfläche des zu operierenden Objekts relativ zur jeweiligen Detektionseinrichtung definiert, wobei die Anordnung/Lage einerseits den Abstand und andererseits die räumliche Orientierung/Ausrichtung in Bezug zur jeweiligen Detektionseinrichtung umfasst. Der Bezugspunkte "Detektionseinrichtung" ist so zu verstehen, dass damit vornehmlich der erfassende Teil der Detektionseinrichtung, d.h. das eigentliche Detektionsmittel, etwa die Sensorik, gemeint ist. Die Detektionseinrichtung ermittelt gemäss der Erfindung somit einerseits den Abstand und andererseits die Orientierung/Ausrichtung des jeweils von ihr erfassten dreidimensionalen Oberflächenbereichs des zu operierenden Objekts relativ zu sich selbst. Die entsprechenden Lagedaten umfassen daher Daten sowohl über den Abstand als auch die Ausrichtung der Aussenkonturdaten.

Das zu operierende Objekt kann grundsätzlich, aber nicht ausschliesslich Knochen-, andere Stützgewebe und/oder Weichteile (etwa pathologische Strukturen, Raumforderungen oder Tumore) umfassen. Im Bereich der Wirbelsäule kann das zu operierende Objekt insbesondere knöcherne und/oder knorpelige Teile der Wirbelsäule selbst umfassen, etwa einen oder mehrere Wirbel, oder einen oder mehrere Teile eines oder mehrerer Wirbel (Wirbelkörper, Querfortsatz, Dornfortsatz, Wirbelbogen, Ansatz für Rippen), das Kreuzbein (Os sacrum), das Steißbein (Os coccygis), und/oder eine oder mehrere Bandscheiben. Das zu operierende Objekt kann im Bereich der Halswirbelsäule ((Pars cervicalis), der Brustwirbelsäule (Pars thoracica) und/oder der Lendenwirbelsäule (Pars lumbalis) liegen. Weiterhin kann das zu operierendes Objekt ein oder mehrere Bänder oder Teile davon umfassen, etwa das vordere Längsband (Ligamentum longitudinale anterius), das hintere Längsband (Ligamentum longitudinale posterius), ein oder mehrere gelbe Bänder (Ligamenta flava), ein oder mehrere Zwischenquerfortsatzbänder (Ligamenta intertransversaria), ein oder mehrere Zwischendornfortsatzbänder (Ligamenta interspinalia) und/oder das Überdornfortsatzband (Ligamentum supraspinale). Das zu operierende Objekt kann auch im an die Wirbelsäule angrenzenden Beckenbereich liegen, also etwa einen angrenzenden Teil des knöchernen Beckens, insbesondere den Beckenring, betreffen. Ebenso kann das zu operierende Objekt im an die Wirbelsäule angrenzenden Thoraxbereich liegen, insbesondere einen oder mehrere Ansätze für Rippen an den Wirbel, eine oder mehrere Rippen(teile) oder sogar - bei massiven pathologischen Veränderungen - das Brustbein betreffen. Ebenso kann das zu operierende Objekt im an die Wirbelsäule angrenzenden Kopfbereich liegen. Dort kann es insbesondere den Bereich des sogenannten C0-Wirbels, also das Hinterhauptbein (Os occipitale) bzw. das Occiput umfassen. Auch kann das zu operierende Objekt sonstiges Weichgewebe, insbesondere nervale Elemente oder pathologische Strukturen, im Bereich der Wirbelsäule und/oder im daran angrenzenden Thorax-, Becken- oder Kopfbereich umfassen.

Gemäss der Erfindung sind die Detektionseinrichtungen jeweils dazu ausgebildet, Bilddaten zu erfassen, die jeweils Informationen über zumindest die Aussenkontur des zu operierenden Objekts bzw. eines Teilbereichs davon umfassen. Je nach Art bzw. Detektionsprinzip der Detektionseinrichtung und je nach Aufbau des zu operierenden Objekts kann eine oder mehrere dieser nicht-röntgenbasierten Detektionseinrichtungen mitunter auch dazu ausbildet sein, neben der Aussenkontur des zu operierenden Objekts auch bildgebende Informationen über die innere Struktur des zu operierenden Objekts zu erfassen. So können etwa auf Ultraschall oder Terahertzstrahlung basierende Detektionseinrichtungen in der Lage sein, je nach Struktur, Aufbau und Gewebe/Material des zu operierenden Objekts über die Aussenkontur hinaus mit einer materialabhängigen Eindringtiefe oder sogar Durchdringung Informationen über die innere Struktur des zu operierenden Objekts zu liefern.

Die erfindungsgemässe Vorrichtung umfasst im Weiteren eine Positionsbestimmungseinrichtung, die dazu ausgebildet ist, in Echtzeit Daten zur jeweiligen Position der Detektionseinrichtungen relativ zu einem stationären Bezugssystem zu ermitteln, vorzugsweise zu einem relativ zum Operationssaal stationären Bezugssystem. Auch hier ist der Bezugspunkte "Detektionseinrichtung" im Hinblick auf die relative Lage zum stationären Bezugssystem so zu verstehen, dass damit vornehmlich der erfassende Teil der Detektionseinrichtung, d.h. das eigentliche Detektionsmittel, etwa die Sensorik, gemeint ist. Mit der Positionsbestimmungseinrichtung als letztes Glied in der Kette zwischen dem zu operierenden Objekt und dem stationären Bezugssystem wird insgesamt gewährleistet, dass die erfassten Aussenkonturdaten der dreidimensionalen Oberfläche jedes Teilbereichs des zu operierenden Objekts über die Lagedaten der Detektionseinrichtungen relativ zum jeweils erfassten Teilbereich und im Weiteren über die Lagedaten der Detektionseinrichtungen relativ zum stationären Bezugsystem räumlich auf das stationären Bezugsystems referenziert werden können. Somit stehen insgesamt Information darüber zur Verfügung, wie die einzelnen erfassten Teilbereiche der dreidimensionalen Oberfläche des zu operierenden Objekts relativ zum stationären Bezugssystem orientiert sind. Erfindungsgemäss ist ferner eine Datenverarbeitungseinrichtung jeweils mit den mehreren Detektionseinrichtungen und der Positionsbestimmungseinrichtung wirkverbunden. Die Datenverarbeitungseinrichtung ist dazu ausgebildet, ein auf das stationäre Bezugssystem referenziertes, virtuelles, dreidimensionales Echtzeit-Bild des zu operierenden Objekts zu erzeugen, und zwar auf Basis der jeweiligen Bild- und Lagedaten der Detektionseinrichtungen sowie auf Basis der jeweiligen Positionsdaten der Positionsbestimmungseinrichtung.

Zur Darstellung des virtuellen Echtzeit-Bildes ist gemäss der Erfindung ferner eine Bildanzeigeeinrichtung vorgesehen, die mit der Verarbeitungseinrichtung wirkverbunden ist.

Gemäss .der Erfindung weist ausserdem wenigstens eine der mehreren Detektionseinrichtungen wenigstens ein Detektionsmittel auf, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen.

Dabei bedeutet "dem Körperinneren zugewandt", dass die entsprechende Aussenkontur der Wirbelsäule oder des an die Wirbelsäule angrenzenden Thorax-, Becken- oder Kopfbereichs während der Operation intrakorporal liegt. Somit kann "dem Körperinneren zugewandt" nicht nur "medial" oder "profund", d.h. zur Mitte des Körpers hin, insbesondere einen "ventral" gelegenen Teilbereich umfassen, sondern auch seitlich, seitlich schräg nach hinten, seitlich schräg nach vorn, hinten schräg nach oben, hinten schräg nach unten, vorn schräg nach oben, vorn schräg nach unten, seitlich schräg nach oben oder seitlich schräg nach unten weisend. Diese Orts- und Richtungsangaben beziehen sich rein definitionshalber auf einen aufrecht stehenden Patienten. Der entsprechende Teilbereich kann während der Operation freipräpariert, teilweise freipräpariert oder vollständig von Gewebe umgeben sein.

Es wurde in erfindungsgemässer Weise erkannt, dass durch die Verwendung mehrerer Detektionseinrichtungen, die um die Operationsstelle herum verteilt sind und von denen im Speziellen wenigstens eine im Inneren des Körpers angeordnet ist, anstelle nur eines begrenzten Teilausschnitts bzw. einer Projektion ein wesentlich umfassenderes Bild des zu operierenden Objekts aus verschiedenen Blickwinkeln erzeugt werden kann, das insbesondere Informationen über zumindest die .Aussenkontur wenigstens eines dem Körperinneren zugewandten Teilbereichs des zu operierenden Objekts enthält. Im Idealfall kann sogar ein virtuelles, dreidimensionales Echtzeit-Rundumbild bzw. eine vollständige Echtzeit-3D-Rekonstruktion der Aussenkontur des zu operierenden Objekts erzeugt werden, was in erfindungswesentlicher Weise die Navigationsgenauigkeit und das Sicherheitsgefühl für den Operateur und letztlich die Sicherheit des Patienten deutlich verbessert.

Insbesondere beruht das mit der erfindungsgemässen Vorrichtung erzeugte virtuelle Echtzeit-Bild auf tatsächlichen Echtzeit-Bilddaten, die nicht nur die Nachverfolgung der Echtzeit-Position des Operationsbereichs relativ zum stationären Bezugssystem erlauben, sondern auch in Echtzeit die tatsächliche Aussenkontur des zu operierenden Objekts quasi in situ bildlich wiedergeben. In vorteilhafter Weise können daher mit der erfindungsgemässen Vorrichtung - im Gegensatz zu Verfahren und Vorrichtungen, bei denen das virtuelle Bild der Operationsstelle abgesehen von den Positionsdaten ausschliesslich auf präoperativen oder während des ersten Teils der Operation, d.h. während der Zugangsphase, genommenen Daten beruhen - intraoperative Veränderungen an der Aussenkontur des zu operierenden Objekts und in der Umgebung der Operationsstelle in Echtzeit bildlich erfasst werden. So werden etwa im Operationsbereich intraoperativ vorgenommene Knochen- oder übrige Gewebeabtragungen, eingebrachte Schrauben, in Ansatz gebrachte Operationswerkzeuge, Stellungskorrekturen/Umstellungen des Alignements oder dergleichen in situ bildlich erfasst, wodurch im Weiteren die Navigationsgenauigkeit und das Sicherheitsgefühl für den Operateur in erfindungswesentlicher Weise gesteigert werden.

Selbstverständlich kann nicht nur eine Detektionseinrichtung dazu ausgebildet sein, um im Inneren des Körpers angeordnet zu werden bzw. intraoperativ angeordnet zu sein, sondern wenigstens zwei, wenigstens drei, wenigstens vier, wenigstens fünf, wenigstens sechs, wenigstens sieben, wenigstens acht, wenigstens neun, wenigstens zehn, wenigstens elf, wenigstens zwölf oder mehr; insbesondere zwei, drei, vier, fünf, sechs, sieben, acht, neun, zehn, elf, zwölf der mehreren Detektionseinrichtungen.

Die Anzahl und Anordnung der Gesamtheit der mehreren Detektionseinrichtungen kann sich nach der Grösse und Lage des zu operierenden Objekts richten. Denkbar ist beispielsweise, dass nur ein kleiner Teil/Abschnitt der Wirbelsäule von der Operation betroffen ist. Hierfür kann bereits eine geringe Anzahl an Detektionseinrichtungen ausreichend sein, die in einem eng begrenzten Bereich um die Operationsstelle herum angeordnet sind. In einem anderen Fall kann die Operation einen wesentlichen grösseren Bereich betreffen, der sich insbesondere über die gesamte Länge der Wirbelsäule erstrecken kann, so dass eine grössere Anzahl von Detektionseinrichtungen notwendig ist, die über die gesamte Länge des Operationsbereichs entlang der Wirbelsäule verteilt angeordnet sind.

Ebenso kann die Art der Detektionseinrichtung je nach Position, insbesondere abhängig von der Anordnung innerhalb oder ausserhalb des Körpers, für die mehreren Detektionseinrichtungen unterschiedlich sein. Denkbar ist beispielsweise, dass eine Detektionseinrichtung, die innerhalb des Körpers angeordnet ist, eine ultraschallbasierte Detektionseinrichtung ist, während eine Detektionseinrichtung der gleichen Vorrichtung, die ausserhalb des Körpers angeordnet ist, ein optischer Konturscanner ist. Durch die Verwendung mehrerer Detektionseinrichtungen unterschiedlicher Art können in vorteilhafter Weise mehr und detailliertere Informationen über das zu operierende Objekt gewonnen werden.

Denkbar ist ferner, dass sich die erfassten Teilbereiche des zu operierenden Objekts, die jeweils unterschiedlichen Detektionseinrichtungen zugeordnet sind, teilweise überlappen, d.h. dass sich die Detektionsfelder unterschiedlicher Detektionseinrichtungen teilweise überlappen.

Die Erzeugung des virtuellen Echtzeit-Bildes kann so erfolgen, dass die einzelnen von den Detektionseinrichtungen erfassten Bilddatensätze, die jeweils wenigstens die Aussenkontur eines Teilbereichs des zu operierenden Objekts wiedergeben, von der Datenverarbeitungseinrichtung zu einem Gesamtbild zumindest der Aussenkontur des zu operierenden Objekts zusammengesetzt werden. Hierfür werden einerseits die von der Detektionseinrichtung bestimmten Daten zur Lage, d.h. Orientierung und Abstand, des entsprechenden Teilbereichs relativ zur jeweiligen Detektionseinrichtung und andererseits die von der Positionsbestimmungseinrichtung erfassten Postionsdaten der jeweiligen Detektionseinrichtung relativ zum stationären Bezugssystem verwendet, um mithilfe dieser Informationen aus den Bilddatensätzen der einzelnen Teilbereiche das Gesamtbild des zu operierenden Objekts zusammenzusetzen. Hierzu kann in der Detektionseinrichtung eine spezielle Bildregistrierungs-, Bildgenerierung- und/oder Bildbearbeitungssoftware zum Einsatz kommen, die aus den Bilddatensätzen den zugeordneten Lage- bzw. Positionsdaten ein virtuelles Bild zumindest der Aussenkontur des zu operierenden Objekts rekonstruiert. Zum räumlich exakten Zusammensetzen der einzelnen Bilddatensätze zu einem Gesamtbild kann die Datenverarbeitungseinrichtung insbesondere dazu ausgebildet sein, spezielle Bildbereiche zu identifizieren, die anatomisch markante Merkmale des zu operierenden Objekts aufweisen, beispielsweise Dornfortsätze, Querfortsätze, Gelenkfortsätze, Rippen, Rippenansätze oder andere markante Merkmale im Wirbelsäulen-, Becken- Thorax oder Occiput-Bereich. Auch Gefässe können als anatomisch markante Merkmale genutzt werden, die sich im Bereich des zu operierenden Objekts befinden, beispielsweise die Arteria vertebralis, die jeweils durch das Foramen transversarium läuft. Vorzugsweise kann die Datenverarbeitungseinrichtung dazu ausgebildet sein, gleiche anatomisch markante Objekte in den Bilddatensätzen, die von verschiedenen Detektionseinrichtungen stammen, zu suchen und anhand der Position und Orientierung dieser anatomisch markanten Objekte die Bilddatensätze der verschiedenen Detektionseinrichtungen räumlich einander zuzuordnen bzw. in der richtigen Weise räumlich zueinander anzuordnen.

Für die Erzeugung des virtuellen Echtzeit-Bilds können insbesondere grundsätzlich bekannte Verfahren der Bildregistrierung zum Einsatz kommen. Beispielsweise kann das Registrierungsverfahren folgende Schritte umfassen: In einem ersten Schritt erfolgt die sogenannte Merkmalsextraktion: Aus den zu registrierenden Bilddatensätzen werden anatomisch markante Merkmale des zu operierenden Objekts, wie z. B. Dorn- oder Querforstätze oder dergleichen, manuell oder automatisch detektiert. In einem zweiten Schritt, der sogenannten Merkmalsanpassung, wird die Korrespondenz der extrahierten Merkmalspunkte hergestellt. Hierzu kann die Detektionseinrichtung beispielsweise eine Funktion verwenden, die ein qualitatives Mass für Übereinstimmungen in den Echtzeit-Bilddatensätzen der verschiedenen Detektionseinrichtungen angibt, welches dann .in einem optimierungsverfahren zu minimieren oder maximieren ist. In einem dritten Schritt erfolgt die Transformationsberechnung, in der ein geeigneter Transformationstyp, z. B. ein affiner Transformationstyp, ein projektiver Transformationstyp oder dergleichen, gewählt und die Transformationsparameter berechnet werden. Schliesslich erfolgt in einem vierten Schritt die eigentliche Transformation. Dazu werden die Bilddatensätzen mit der im vorherigen Schritt berechneten Umbildung transformiert, wobei mitunter auch Interpolationstechniken zum Einsatz kommen können.

Nach einer ersten vorteilhaften Ausgestaltung der Erfindung können neben den Echtzeit-Bilddaten präoperativ oder während der Zugangsphase gewonnene Bilddaten des zu operierenden Objekts mit in die Erzeugung des virtuellen Echtzeit-Bildes integriert werden. Daher kann es insbesondere vorgesehen sein, dass die Datenverarbeitungseinrichtung dazu ausgebildet ist, präoperativ oder während der Zugangsphase gewonnene Bilddaten des zu operierenden Objekts mit den Bilddaten der Detektionseinrichtungen zu überlagern, um auf Basis der mit den Bilddaten der Detektionseinrichtungen überlagerten, präoperativ oder während der Zugangsphase gewonnenen Bilddaten das virtuelle auf das stationäre Bezugssystem referenzierte Echtzeit-Bild des zu operierenden Objekts zu erzeugen. Auch hierbei können wiederum grundsätzlich bekannte Bildregistrierungsverfahren zum Einsatz kommen. Durch die zusätzliche Einbindung präoperativ gewonnener Bilddaten können in vorteilhafter Weise mehr und detailliertere Informationen über den zu operierenden Objekts gewonnen werden.

Grundsätzlich ist aber auch denkbar, dass das virtuelle Echtzeit-Bild im Hinblick auf die graphischen Wiedergabe des zu operierenden Objekts nur auf Basis der mit den Aussenkonturdaten überlagerten präoperativ gewonnenen Bilddaten erzeugt wird, während die intraoperativ gewonnenen Bilddaten, Lagedaten und Positionsdaten nur zur Echt-Referenzierung dieses virtuellen Echtzeit-Bildes auf das stationäre Bezugssystem verwendet werden. Insoweit wird nach einem unabhängigen Gedanken der Offenbarung eine weitere Vorrichtung für die intraoperative, bildgesteuerte Navigation bei chirurgischen Eingriffen im Bereich der Wirbelsäule und/oder im daran angrenzenden Thorax-, Becken- oder Kopfbereich vorgeschlagen, die wenigstens eine vorzugsweise nicht-röntgenbasierte Detektionseinrichtung umfasst, die dazu ausgebildet ist, intraoperativ in Echtzeit Bild- und Lagedaten zu erfassen, die Informationen über zumindest die Aussenkontur wenigstens eines Teilbereichs des zu operierenden Objekts sowie über die Lage des Teilbereichs relativ zur Detektionseinrichtung umfassen. Die Vorrichtung umfasst darüber hinaus eine Positionsbestimmungseinrichtung, dazu ausgebildet ist, in Echtzeit Daten zur Position der wenigstens einen Detektionseinrichtung relativ zu einem stationären Bezugssystem zu ermitteln. Ferner umfasst die Vorrichtung eine mit der wenigstens einen Detektionseinrichtung und der Positionsbestimmungseinrichtung wirkverbundene Datenverarbeitungseinrichtung, die dazu ausgebildet ist, präoperativ oder während der Zugangsphase gewonnene Bilddaten des zu operierenden Objekts mit den Bilddaten der wenigstens einen Detektionseinrichtung zu überlagern, um auf Basis der mit den Bilddaten der Detektionseinrichtung überlagerten, präoperativ oder während der Zugangsphase gewonnenen Bilddaten das virtuelle auf das stationäre Bezugssystem referenzierte Echtzeit-Bild des zu operierenden Objekts zu erzeugen. Auch hierbei können wiederum grundsätzlich bekannte Bildregistrierungsverfahren zum Einsatz kommen. Zur Darstellung des virtuellen Echtzeit-Bildes ist im Weiteren eine mit der Datenverarbeitungseinrichtung wirkverbundene Bildanzeigeeinrichtung vorgesehen. Auch bei dieser Vorrichtung weist die wenigstens eine Detektionseinrichtung wenigstens ein Detektionsmittel auf, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen. Insoweit dient die wenigstens eine Detektionseinrichtung vornehmlich der Nachverfolgung der Echtzeit-Position des Operationsbereichs relativ zum stationären Bezugssystem, indem sie wenigstens einen dem Körperinneren zugewandten Teilbereich des zu operierenden Objekts erfasst und als Referenzbereich zur Überlagerung mit den präoperativ oder während der Zugangsphase gewonnenen Bilddaten.

Die Vorrichtung nach Anspruch 1 beruht somit auf dem Erfindungsgedanken, dass (die) wenigstens eine Detektionseinrichtung bzw. ein Detektionsmittel dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts umfassen. In erfindungsgemässer Weise wurde erkannt, dass die Anordnung der Detektionseinrichtung bzw. des Detektionsmittels im Körperinneren die gesamte Navigation unempfindlich gegen intraoperative Störeinwirkungen macht, so dass stets eine hinreichende Navigationsgenauigkeit gewährleistet ist. Durch die Anordnung im Inneren des Körpers besteht insbesondere eine wenn nur sehr geringe Gefahr, dass das Detektionsfeld der Detektionseinrichtung vom Operateur gestört wird. Umgekehrt beeinträchtigt die wenigstens eine im Körperinneren anordenbare Detektionseinrichtung in vorteilhafter Weise auch nicht die Zugänglichkeit des Operationsbereichs für den Operateur. Des Weiteren erlauben es Detektionsmittel im Inneren des Körpers, andere bildgebende Modalitäten zu benutzen als beispielsweise Röntgenstrahlung, etwa wie beispielsweise Ultraschall. Ultraschall hat insbesondere im Bereich der Halswirbelsäule grosse Vorteile. So kann durch beispielsweise mit entsprechend angepassten Geräten, wie z.B. Geräten für die transösophageale Echokardiographie, die Halswirbelsäule von vorne abgebildet werden, wodurch wichtige Strukturen identifiziert werden können (etwa die Arteria vertebralis oder Nervenwurzeln). Gleichzeitig können die Bewegungen der Vertebrae (Wirbel), die aufgrund der grossen Mobilität der Halswirbelsäule durch chirurgische Manipulation oder sonstige Manöver verursacht werden, kontinuierlich kompensiert werden. Zudem erlauben Detektionsmittel im Inneren des Körpers, andere Aspekte des zu operierenden Objektes darzustellen, die nicht bereits über den Zugangsweg zum zu operierenden Objekt einsehbar sind. Dies führt im Weiteren zu einer Erhöhung der Genauigkeit.

Von Bedeutung ist ebenso, dass die im Körperinneren anordenbare Detektionseinrichtung aufgrund der aktiven Positionsbestimmung mit Hilfe der Positionsbestimmungseinrichtung nicht fixiert werden muss. Vielmehr kann sie sich frei bewegen, sodass die Navigation unempfindlich gegenüber intraoperativen Störeinwirkungen ist, etwa Manipulation am Patient durch den Operateur, Umlagerungen, Atem- oder Darmbewegungen etc. Denn die Positionsbestimmungseinrichtung erfasst gemäss der Erfindung kontinuierlich einerseits die jeweilige Lage der Detektionseinrichtungen relativ zum stationären Bezugssystem. Andererseits überwacht die Detektionseinrichtungen selbst in Echtzeit permanent ihre Lage relativ zu dem von ihnen erfassten Teilbereich der Operationsstelle. Hierdurch wird ermöglicht, die erfassten Aussenkonturdaten und folglich das virtuelle Echtzeit-Bild auf das stationäre Bezugssystem in Echtzeit zu referenzieren.

Gemäß der Erfindung sind die wenigstens zwei im Inneren des Körpers anordenbaren Detektionseinrichtungen jeweils eine ultraschallbasierte Detektionseinrichtung oder eine auf Terahertzstrahlung basierte Detektionseinrichtung. Ultraschallbasierte Detektionseinrichtungen sind in besondere Weise für die Bildgebung in Echtzeit geeignet.

Ferner erlauben ultraschallbasierte Detektionseinrichtungen, die Aussenkontur des zu operierenden Objekts durch Weichgewebe hindurch zu erfassen, also etwa durch Fettgewebe, Muskelgewebe und/oder Bindegewebe. Daher eignen sie sich insbesondere zur Bilderfassung solcher Teilbereiche des zu operierenden Objekts, die intraoperativ nicht freipräpariert werden. Darüber hinaus sind ultraschallbasierte Detektionseinrichtungen mitunter dazu ausgebildet, eine Abstands- und Lagemessung zwischen Detektionseinrichtung und erfasstem Objekt sowie eine räumliche Vermessung des zu operierenden Objekts zu ermöglichen, so dass die Lage des erfassten Teilbereichs relativ zur Detektionseinrichtung sehr genau in Echtzeit erfasst werden kann. Ultraschallbasierte Detektionseinrichtungen erlauben zudem Doppler-sonografische Messungen von Flüssigkeitsströmungen. So können z.B. wichtige Blutgefässe als Gefahrenzonen oder anatomische Landmarken identifiziert werden. Auch können Flüssigkeitsströmungen im Liquorraum detektiert werden, die zwar zumeist minimal, aber dennoch im Hinblick auf die Operation von grösster Relevanz sind.

Denkbar ist auch, dass die wenigstens eine im Inneren des Körpers anordenbare Detektionseinrichtung eine auf Terahertzstrahlung basierte Detektionseinrichtung ist. Der große Brechungsindex von organischem Gewebe im Terahertz-Spektrum erlaubt sehr kontrastreiche Aufnahmen und kann konventionelle Aufnahmetechniken ergänzen. Die Strahlung ist nicht-ionisierend und kann gefahrlos für medizinische und biologische Anwendungen eingesetzt werden. Terahertzstrahlung vermag u.U. sogar je nach Material/Gewebe Informationen über die innere Struktur des zu operierenden Objekts zu liefern. Information über die innere Struktur können beispielsweise helfen, zwischen gesunden Zellen und Tumorzellen zu unterscheiden

Im Speziellen kann es nach einer weiteren Ausgestaltung der Erfindung vorgesehen sein, dass die wenigstens eine im Inneren des Körpers anordenbare Detektionseinrichtung dazu ausgebildet ist, über eine präformierte Körperhöhle, insbesondere die Nase, den Mund, den Rachenraum, die Speiseröhre, die Luftröhre, den Magen-Darmtrakt, die Harnblase, oder über ein Blutgefäss, in den Körper eingebracht zu werden. Für solche Applikationen eignen sich insbesondere endoskopische Detektionseinrichtungen für die Endosonografie, etwa für die gastroenterologische Endosonografie. Die Lage/Position letztgenannter Detektionsrichtungen für die gastroenterologische Endosonografie kann entweder direkt über das Endoskop oder etwa mittels elektromagnetischer Wellen (Funk) nach aussen erfolgen. Für die Positionsbestimmung können insbesondere Lagesensoren in Betracht kommen, die mitunter in der Lage sind, am Endoskop auftretende Beschleunigung zu messen, um daraus auf die Lage und Position des Detektionsmittels zu schliessen.

Ferner kann es vorgesehen sein, dass die wenigstens eine im Inneren des Körpers anordenbare Detektionseinrichtung dazu ausgebbildet ist, operativ in ein Gewebe, beispielsweise in paravertebrales Muskelgewebe oder Muskelgewebe des Gesässmuskels, und/oder in ein Fettgewebe, beispielsweise den Epiduralraum, eingebracht zu werden. Für solche Applikationen eignen sich neben den oben beschriebenen, konventionellen Endosonografie-Sonden insbesondere sogenannte sonografische Minisonden-Systeme, die etwa durch einen Biopsiekanal geschoben werden können und eine wesentlich dünnere Sonde als bei der konventionellen Endosonografie aufweisen.

Analog zu der wenigstens einen im Inneren des Körpers anordenbaren Detektionseinrichtung kann wenigstens eine der mehreren Detektionseinrichtungen dazu ausgebildet sein, ausserhalb des Körpers angeordnet zu werden, um Bilddaten zu erfassen, die Informationen über zumindest die Aussenkontur eines dem Körperäusseren zugewandten Teilbereichs des zu operierenden Objekts umfassen.

In gleicher Weise kann es ferner vorgesehen sein, dass die wenigstens eine ausserhalb des Körpers anordenbare Detektionseinrichtung eine ultraschallbasierte Detektionseinrichtung oder eine auf Terahertzstrahlung basierte Detektionseinrichtung ist. Denkbar ist aber auch, dass die wenigstens eine ausserhalb des Körpers anordenbare Detektionseinrichtung als ein optischer Konturscanner ausgebildet ist.

Als Positionsbestimmungseinrichtungen kommen beispielsweise elektromagnetische, insbesondere optische Positionsbestimmungseinrichtungen in Betracht, bei denen an den Detektionseinrichtungen jeweils ein passiver Positionsmarker oder ein aktiver Positionssender angeordnet ist, der über eine entfernt angeordnete Positionsmarker-Erkennungseinrichtung oder eine entfernt angeordnete Empfängereinrichtung, die von dem Positionssender ausgesandte Signale empfängt, überwacht wird. Solche Positionsbestimmungseinrichtungen sind grundsätzlich aus dem Stand der Technik bekannt, beispielsweise die für die Spinalchirurgie unter dem Markennamen "StealthStation" vertriebene Positionsbestimmungseinrichtung von Medtronic.

Um dem Operateur - wie zuvor erläutert - auch Echtzeit-Informationen zu etwaigen Operationswerkzeugen und deren Position und Orientierung relativ zur Operationsstelle intraoperativ zur Verfügung zu stellen, kann es nach einer weiteren Ausgestaltung der Erfindung vorgesehen sein, dass die Vorrichtung zum einen wenigstens ein Operationswerkzeug umfasst, und dass zum anderen die Positionsbestimmungseinrichtung ferner zur Ermittlung der Lage des wenigstens einen Operationswerkzeuges relativ zum stationären Bezugsystem ausgebildet ist und die Verarbeitungseinheit ferner zum Einblenden eines Indikators in das virtuelle Echtzeit-Bild ausgebildet ist, der das wenigstens eine Operationswerkzeug und dessen Lage relativ zum stationären Bezugsystem repräsentiert.

Darüber hinaus kann es bei einer weiteren vorteilhaften Ausgestaltung der Erfindung vorgesehen sein, dass wenigstens eine der Detektionseinrichtungen an einem Operationswerkzeug anordenbar ist bzw. angeordnet ist. Hierzu kann die erfindungsgemässe Vorrichtung ferner ein Operationswerkzeug umfassen, das für die Aufnahme wenigstens einer Detektionseinrichtung ausgebildet ist bzw. an dem wenigstens eine Detektionseinrichtung angeordnet ist.

Um die Funktionalität der Vorrichtung gegen etwaige äussere Störeinflüsse abzusichern und somit das Sicherheitsgefühl für den Operateur konstant aufrecht zu erhalten, kann es nach einer weiteren vorteilhaften Ausgestaltung der Erfindung vorgesehen sein, dass die Datenverarbeitungseinrichtung dazu ausgebildet ist, bei einem temporären Ausfall der Bilddaten-, der Lagedaten und/oder der Positionsdatenermittlung wenigstens einer der Detektionseinrichtungen den von dieser Detektionseinrichtung erfassten Teilbereich des zu operierenden Objekts in dem virtuellen Echtzeit-Bild auf Basis der zu einem früheren Zeitpunkt von dieser Detektionseinrichtung bei funktionierender Lagedaten- und Positionsdatenermittlung erfassten Bilddaten zu erzeugen, wobei die zu einem früheren Zeitpunkt erfassten Bilddaten des entsprechenden Teilbereichs in Echtzeit an die aktuelle Lage der von den übrigen Detektionseinrichtungen erfassten Teilbereiche anpasst werden.

Für den Fall, dass lediglich die Lagedaten- und/oder Positionsdatenermittlung für eine oder mehrere der Detektionseinrichtungen temporär ausfällt, nicht jedoch die Bilderfassung der betroffene(n) Detektionseinrichtung(en), kann es nach einer weiteren Ausgestaltung der Erfindung vorgesehen sein, den von dieser Detektionseinrichtung erfassten Teilbereich des zu operierenden Objekts in dem virtuellen Echtzeit-Bild zwar auf Basis der aktuell erfassten Bilddaten zu erzeugen, jedoch die aktuell erfassten Bilddaten des entsprechenden Teilbereichs im Hinblick auf die Lage relativ zum stationären Bezugssystem in Echtzeit an die aktuelle Lage der von den übrigen Detektionseinrichtungen erfassten Teilbereiche anzupassen.

Ferner kann es vorgesehen sein, dass die Detektionseinrichtungen dazu ausgebildet sind, miteinander zu kommunizieren, insbesondere Informationen über ihre jeweilige Position und/oder Lage, vorzugsweise relativ zum stationären Bezugssystem, auszutauschen. Darüber hinaus kann es vorgesehen sein, dass wenigstens eine der mehreren Detektionseinrichtungen, insbesondere wenigstens ein Detektionsmittel wenigstens einer der Detektionseinrichtungen, dazu ausgebildet sind, die Position und/oder Lage wenigstens einer anderen Detektionseinrichtung bzw. die Position und/oder Lage des jeweiligen Detektionsmittels der wenigstens einen anderen Detektionseinrichtung relativ zu sich und damit über die Positionsbestimmungseinrichtung relativ zum stationären Bezugssystem zu ermitteln. Hierdurch kann die Navigationsgenauigkeit im Weiteren deutlich verbessert werden. Denkbar ist beispielsweise, dass eine der mehreren Detektionseinrichtungen eine Ultraschall-Detektionseinrichtung ist, die nicht nur dazu ausgebildet ist, Bild- und Lagedaten über wenigstens einen Teilbereich des zu operierende Objekts zu erfassen, sondern auch wenigstens Lage- und Postionsdaten, vorzugsweise auch Bilddaten, zu wenigstens einer anderen Detektionseinrichtung relativ zu sich zu erfassen.

Um zusätzlich weitere Informationen über die Aussenkontur und insbesondere die innere Struktur des zu operierenden Objekts zu erhalten, kann es nach einer weiteren Ausgestaltung der Erfindung vorgesehen sein, dass die Vorrichtung ferner wenigstens eine röntgenbasierte Detektionseinrichtung aufweist, die dazu ausgebildet ist, intraoperativ in Echtzeit Bilddaten zu erfassen, die Informationen über die Aussenkontur und innere Struktur wenigstens eines Teilbereichs des zu operierenden Objekts umfassen, wobei die Datenverarbeitungseinrichtung mit der röntgenbasierten Detektionseinrichtung wirkverbunden ist und dazu ausgebildet ist, die Bilddaten der röntgenbasierten Detektionseinrichtung mit den Bilddaten der nicht-röntgenbasierten Detektionseinrichtungen zu überlagern, um auf Basis der miteinander überlagerten Bilddaten das virtuelle auf das stationäre Bezugssystem referenzierte Echtzeit-Bild des zu operierenden Objekts zu erzeugen, das Informationen über die Aussenkontur und innere Struktur des zu operierenden Objekts umfasst. Denkbar ist auch, dass zusätzlich präoperativ oder während der Zugangsphase gewonnene Bilddaten - wie weiter oben beschrieben - mit zuvor genannten intraoperativen Daten der röntgenbasierten Detektionseinrichtung und nicht-röntgenbasierten Detektionseinrichtungen überlagert werden. Nach einer weiteren Ausgestaltung der Erfindung kann es auch vorgesehen sein, dass die Vorrichtung ferner wenigstens eine röntgenbasierte Detektionseinrichtung aufweist, die dazu ausgebildet ist, intraoperativ in Echtzeit Bild- und Lagedaten zu erfassen, die Informationen über die Aussenkontur und innere Struktur wenigstens eines Teilbereichs des zu operierenden Objekts sowie über die Lage des Teilbereichs relativ zur röntgenbasierten Detektionseinrichtung umfassen, wobei die Positionsbestimmungseinrichtung ferner dazu ausgebildet ist, in Echtzeit Daten zur Position der röntgenbasierten Detektionseinrichtung relativ zum stationären Bezugssystem zu ermitteln, und wobei die Datenverarbeitungseinrichtung mit der röntgenbasierten Detektionseinrichtung wirkverbunden ist und dazu ausgebildet ist, auf Basis der jeweiligen Bild- und Lagedaten der nicht-röntgenbasierten Detektionseinrichtungen, der Bild- und Lagedaten der röntgenbasierten Detektionseinrichtung und der jeweiligen Positionsdaten der Positionsbestimmungseinrichtung ein auf das stationäre Bezugssystem referenziertes, virtuelles, dreidimensionales Echtzeit-Bild des zu operierenden Objekts zu erzeugen, das Informationen über die Aussenkontur und innere Struktur des zu operierenden Objekts umfasst.

Vorzugsweise ist die röntgenbasierten Detektionseinrichtung möglichst schwach strahlend bzw. niedrig dosiert, um die Strahlenbelastung für den Patienten und das Operationspersonal so gering wie möglich zu halten.

Die gemäss der Offenbarung vorgesehene Bildanzeigeeinrichtung zur Darstellung des virtuellen Echtzeit-Bildes kann beispielsweise als stationärer Monitor im Operationssaal ausgebildet sein. Denkbar ist aber auch, dass die Bildanzeigeeinrichtung als ein tragbarer Monitor ausgebildet ist, der insbesondere am Handgelenk des Operateurs oder am Kopf vor den Augen des Operateurs oder an einem Instrument/Werkzeug angeordnet werden kann.

Weitere Einzelheiten der Erfindung und insbesondere eine beispielhafte Ausführungsform der vorgeschlagenen Vorrichtung werden im Folgenden anhand der beigefügten Zeichnungen erläutert.

In den Zeichnungen zeigen:
- Fig. 1: eine schematische Ansicht eines möglichen Ausführungsbeispiels der erfindungsgemässen Vorrichtung,
- Fig. 2: eine Detailansicht der erfindungsgemässen Vorrichtung gemäss Fig. 1, und
- Fig. 3: eine schematische Ansicht einer weiteren möglichen Anordnung der erfindungsgemässen Vorrichtung.

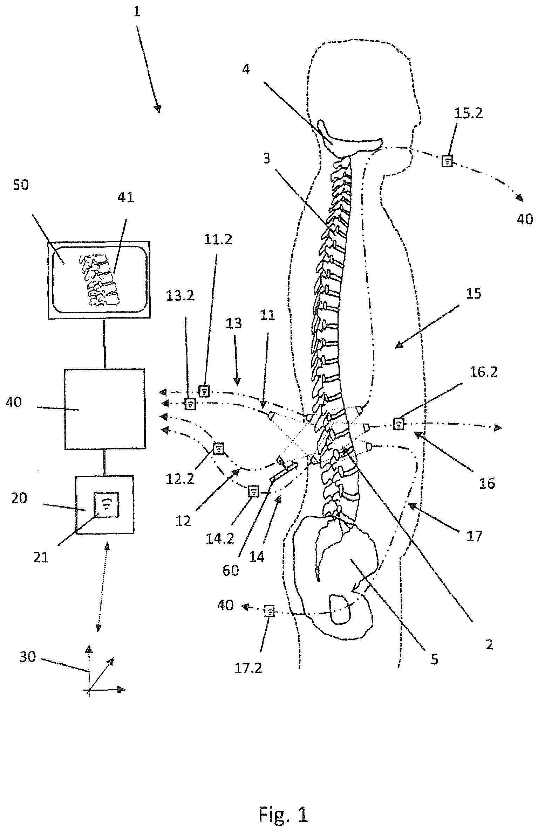

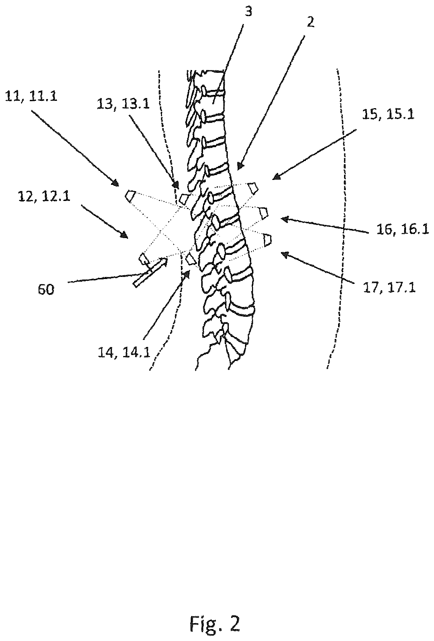

Die Fig. 1 und 2 zeigen ein mögliches Ausführungsbeispiel der erfindungsgemässen Vorrichtung für die intraoperative, bildgesteuerte Navigation bei chirurgischen Eingriffen im Bereich der Wirbelsäule 3 und/oder im daran angrenzenden Thorax-, Becken- 5 oder Kopfbereich 4.

Die Vorrichtung 1 umfasst mehrere nicht-röntgenbasierte Detektionseinrichtungen 11-17, die jeweils dazu ausgebildet sind, um ein zu operierenden Objekt 2 an der Wirbelsäule 3 eines Patienten verteilt angeordnet zu werden. Jede dieser Detektionseinrichtungen 11-17 ist ferner dazu ausgebildet, intraoperativ jeweils zumindest die Aussenkontur wenigstens eines Teilbereichs des zu operierenden Objekts 2 sowie die Lage dieses Teilbereichs relativ zur jeweiligen Detektionseinrichtung 11-17 in Echtzeit zu erfassen.

Wie den Fig. 1 und 2 zu entnehmen ist, sind die Detektionseinrichtungen 11 und 12 bzw. die ihnen zugeordneten Detektionsmittel 11.1 und 12.1 ausserhalb des Körpers angeordnet, während die Detektionseinrichtungen 13-17 bzw. die ihnen zugeordneten Detektionsmittel 13.1-17.1 innerhalb des Körpers angeordnet sind. Im vorliegenden Ausführungsbeispiel sind die Detektionseinrichtungen 13 und 14 bzw. die ihnen zugeordneten Detektionsmittel 13.1 und 14.1 operativ in das paravertebrale Muskelgewebe um den zu operierenden freipräparierten Knochenbereich 2 eingebracht. Ebenso ist das Detektionsmittel 16.1 der Detektionseinrichtung 16 operativ über die Bauchhöhle an die Vorderseite des zu operierenden Knochenbereichs 2 der Wirbelsäule 3 eingebracht.

Demgegenüber ist das Detektionsmittel 15.1 der Detektionseinrichtung 15 als Endoskop ausgebildet und über den Mund, den Rachenraum und die Speiseröhre in den Magen eingebracht, um von dort aus die Aussenkontur eines Teilbereichs des zu operierenden Knochenbereichs 2 aus Richtungen des Magens oder der Speiseröhre zu erfassen. In ähnlicher Weise ist auch das Detektionsmittel 17.1 der Detektionseinrichtung 17 als Endoskop ausgebildet und rektal in den Dickdarm eingebracht, um von dort aus die Aussenkontur eines anderen Teilbereichs des zu operierenden Knochenbereichs 2 aus dem Körperinneren heraus zu erfassen.

Vorzugsweise sind im vorliegenden Ausführungsbeispiel alle innerhalb des Körpers eingebrachten Detektionseinrichtungen 13 bis 17 bzw. die ihnen zugeordneten Detektionsmittel 13.1-17.1 als ultraschallbasierte Detektionseinrichtungen bzw. Detektionsmittel ausgebildet. Die beiden Detektionsmittel 13.1 und 14.1 sind im vorliegenden Ausführungsbeispiel als sogenannte Minisonden-Systeme ausgebildet, die durch einen jeweiligen Biopsiekanal in das paravertebrale Muskelgewebe eingeschoben werden können. Demgegenüber sind die Detektionsmittel 15.1 und 17.1 als endoskopische Ultraschallsonden ausgebildet.

Die beiden ausserhalb des Körpers angeordneten Detektionseinrichtungen 11 und 12 bzw. die ihnen zugeordneten Detektionsmittel 11.1 und 12.1 sind im vorliegenden Ausführungsbeispiel als optische Konturscanner ausgebildet, die jeweils einen Teilbereich der Aussenkontur des zu operierenden Knochenbereichs 2 optisch mittels Laserlicht abtasten.

Die in den Fig. 1 und 2 gestrichelt gezeichneten Linien, die von den jeweiligen Detektionsmitteln 11.1-17.1 ausgehen, repräsentieren jeweils das entsprechende Detektionsfeld der jeweiligen Detektionseinrichtung 11-17. Wie zu erkennen ist, können sich die Detektionsfelder verschiedener Detektionseinrichtungen 11-17 überlappen. Im vorliegenden Ausführungsbeispiel überlappen sich die Detektionsfelder insbesondere so, dass eine nahezu vollständige Rundum-Darstellung des zu operierenden Knochenbereichs 2 ermöglicht wird.

Alle Detektionseinrichtungen 11-17 sind ferner dazu ausgebildet, die Lage des jeweils erfassten Teilbereichs zu sich selbst zu erfassen. Hierzu eignen sich insbesondere ultraschallbasierte Detektionseinrichtungen und optische Konturscanner. Dies ist eine Grundvoraussetzung dafür, die erfassten Aussenkonturdaten überhaupt in Bezug zu einem stationären Bezugssystem zu setzen.

Um die jeweiligen Lagen/Positionen der Detektionseinrichtungen 11-17 in Bezug auf ein stationäres Bezugssystem 30, etwa ein bezüglich des Operationssaals stationäres Bezugssystem 30, zu ermitteln, weist die erfindungsgemässe Vorrichtung 1 ferner eine Positionsbestimmungseinrichtung 20 auf. Mit der Lage/Position der Detektionseinrichtungen 11-17 ist vornehmlich die Lage/Position der jeweiligen Detektionsmittel 11.1-17.1 in Bezug auf das stationäre Bezugssystem 30 gemeint.

Im vorliegenden Ausführungsbeispiel ist die Positionsbestimmungseinrichtung 20 als elektromagnetisches Positionserkennungssystem ausgebildet. Dieses System umfasst einen an jedem der Detektionseinrichtungen passiven Positionsmarker 11.2, 12.2 oder einen Positionssender 13.2-17.2, der über eine entfernt angeordnete, kombinierte Positionsmarker-Erkennungseinrichtung und Positionssender-Empfängereinrichtung 21 überwacht wird. Diese kombinierte Einrichtung ist dazu ausgebildet, in Echtzeit einerseits die räumliche Position der Positionsmarker 11.2 und 12.2 und damit die räumliche Position der Detektionseinrichtungen 11 und 12 zu erkennen und zu verfolgen und andererseits die von den Positionssendern 13.2-17.2 ausgesandten Positionssignale zu empfangen und daraus eine räumliche Position der zu geordneten Detektionseinrichtungen 13-17 zu bestimmen.

Für die Erkennung müssen die Positionsmarker 11.2 und 12.2 typischerweise ausserhalb des Körpers angeordnet sein.

Demgegenüber können die Positionssender 13.2-17.2 sowohl innerhalb als auch ausserhalb des Körpers angeordnet sein. Im vorliegenden Ausführungsbeispiel ist insbesondere der Positionssender 16.2 dazu ausgebildet, innerhalb des Körpers angeordnet zu werden und von dort aus Positionssignale, die Informationen über die Lage des Detektionsmittel 16.1, nach aussen zu senden, so dass sie dort von der Positionssender-Empfängereinrichtung 21 empfangen werden können. Im vorliegenden Ausführungsbeispiel sind sämtliche Positionssender 13.2-17.2 dazu ausgerichtet, drahtlos die jeweiligen Positionssignale an die Positionssender-Empfängereinrichtung 21 zu übertragen. Denkbar ist aber auch, dass die Übertragung der Positionssignale bei einem oder mehreren der Positionssender drahtgebunden erfolgt.

Die von der Positionsbestimmungseinrichtung 20 bezüglich des stationären Bezugssystems 30 ermittelten Lagedaten der Detektionseinrichtungen 13-17 bzw. der Detektionsmittel 13.1-17.1 sowie die von den Detektionsmitteln 13.1-17.1 bzw. den Detektionseinrichtungen 13-17 ermittelten Lagedaten relativ zum aussenkonturmässig erfassten Teilbereichs des zu operierenden Knochenbereichs 2 werden einer Datenverarbeitungseinrichtung 40 zur Verfügung gestellt.

Ferner übermitteln die Detektionseinrichtungen 13-17 an die Datenverarbeitungseinrichtung 40 jeweils Bilddaten, die die entsprechenden, von ihnen in Echtzeit erfassten, Aussenkonturen bzw. Aussenkonturdaten des jeweiligen Teilbereichs des zu operierenden Knochenbereichs 2 repräsentieren. Zum Zweck dieses Datentransfers sind die Detektionseinrichtungen 13-17 sowie die Positionsbestimmungseinrichtung 20 mit der Verarbeitungseinrichtung 40 wirkverbunden. Dieser Datentransfer kann beispielsweise über eine drahtgebundene Datenverbindung oder über eine drahtlose Datenverbindung erfolgen.

Die Datenverarbeitungseinrichtung 40 ist im Weiteren dazu ausgebildet, auf Basis der übermittelten Bild- und Lagedaten ein auf das stationäre Bezugssystem 30 referenziertes, virtuelles, dreidimensionales Echtzeit-Bild 41 des zu operierenden Knochenbereichs zu erzeugen, das aus den einzelnen Bilddatensätzen der jeweiligen Teilbereiche zusammengesetzt ist. Dieses virtuelle Echtzeit-Bild beruht in erfindungsgemässer Weise vollständig auf tatsächlichen Echtzeit-Bilddaten, die nicht nur die Nachverfolgung der Echtzeit-Position des Operationsbereichs relativ zum stationären Bezugssystem 30 erlauben, sondern auch in Echtzeit wenigstens die tatsächliche Aussenkontur des zu operierenden Knochenbereichs 2 quasi in situ bildlich wiedergeben. Aufgrund der mehreren innerhalb des Körpers angeordneten Detektionseinrichtungen 13-17 kann in erfindungsgemässer Weise ein im Vergleich zum Stand der Technik wesentlich umfassenderes Bild des zu operierenden Knochenbereichs erzeugt werden. Aufgrund der in Richtung Rückenseite erfassenden Detektionseinrichtungen 15-17 können insbesondere Informationen über die Aussenkontur zumindest eines dem Körperinneren zugewandten Teilbereichs des zu operierenden Knochenbereichs enthalten werden. Im Idealfall kann sogar ein virtuelles Echtzeit-Rundumbild der Aussenkontur des zu operierenden Knochenbereichs erzeugt werden, wodurch die Navigationsgenauigkeit und das Sicherheitsgefühl für den Operateur erheblich verbessert wird.

Das von der Verarbeitungseinrichtung 40 erzeugte virtuelle Echtzeit-Bild 41 kann auf einer damit wirkverbundenen Bildanzeigeeinrichtung 50 dargestellt werden. In vorteilhafter Weise ist diese Bildanzeigeeinrichtung 50 im vorliegenden Ausführungsbeispiel als tragbarer Monitor bzw. als tragbares Display ausgebildet, das insbesondere am Handgelenk des Operateurs oder in Art einer Monitorbrille vor den Augen des Operateurs oder an einem Operationswerkzeug/-instrument befestigt werden kann.

Im vorliegenden Ausführungsbeispiel umfasst die Vorrichtung 1 zudem wenigstens ein Operationswerkzeug 60, das - ähnlich wie die Detektionseinrichtungen 13-17 - mit einem Positionssender oder einem Positionsmarker ausgestattet ist, mit dessen Hilfe die räumliche Lage des Operationswerkzeuges 60 relativ zum stationären Bezugssystem 30 und damit zum zu operierenden Knochenbereich 2 bestimmt werden kann. Zu diesem Zwecke ist die Positionsbestimmungseinrichtung 20 ferner zur Ermittlung der Lage des wenigstens einen Operationswerkzeuges relativ zum stationären Bezugsystem 30 ausgebildet. Ferner kann die Verarbeitungseinheit 40 zum Einblenden eines Indikators in das virtuelle Echtzeit-Bild 41 ausgebildet sein, der das wenigstens eine Operationswerkzeug 60 und dessen Lage relativ zum stationären Bezugsystem 30 repräsentiert. Hierdurch werden zusätzlich die Navigationsgenauigkeit und das Sicherheitsgefühl für den Operateur verbessert.

Wie insbesondere in Fig. 2 gezeigt ist, kann es vorgesehen sein, dass wenigstens eine der Detektionseinrichtungen - im vorliegenden Ausführungsbeispiel die Detektionseinrichtung 12 - an dem Operationswerkzeug 60 angeordnet ist. Hierzu ist das Operationswerkzeug 60 im Speziellen mit einer Halterung ausgebildet, um die Detektionseinrichtung 12 daran zu befestigen.

Um die Funktionalität der Vorrichtung gegen etwaige äussere Störeinflüsse abzusichern und somit das Sicherheitsgefühl für den Operateur konstant aufrecht zu erhalten, ist die Verarbeitungseinrichtung 40 ferner dazu ausgebildet, bei einem temporären Ausfall der Bilderfassung und/oder der Lage-/Positionsermittlung einer Detektionseinrichtung 11-17 den von dieser Detektionseinrichtung 11-17 erfassten Teilbereich des zu operierenden Knochenbereichs 2 in dem virtuellen Echtzeit-Bild 41 auf Basis der zu einem früheren Zeitpunkt von dieser Detektionseinrichtung 11-17 bei funktionierender Lageermittlung erfassten Bilddaten zu erzeugen. Dazu kann es insbesondere vorgesehen sein, dass die zu einem früheren Zeitpunkt erfassten Bilddaten des entsprechenden Teilbereichs in Echtzeit an die aktuelle Lage der von den übrigen Detektionseinrichtungen 11-17 erfassten Teilbereiche anpasst werden.

Für den Fall, dass lediglich die Lage-/Positionsermittlung für eine oder mehrere der Detektionseinrichtungen 11-17 temporär ausfällt, nicht jedoch die Bilderfassung der betroffenen Detektionseinrichtung(en) 11-17 selbst, kann es ferner vorgesehen sein, den von der jeweils betroffenen Detektionseinrichtung 11-17 erfassten Teilbereich des zu operierenden Knochenbereichs 2 in dem virtuellen Echtzeit-Bild 41 auf Basis der aktuell erfassten Bilddaten zu erzeugen. Hierzu werden die aktuell erfassten Bilddaten des entsprechenden Teilbereichs im Hinblick auf ihre Lage relativ zum stationären Bezugssystem 41 in Echtzeit an die aktuelle Lage der von den übrigen Detektionseinrichtungen erfassten Teilbereiche angepasst.

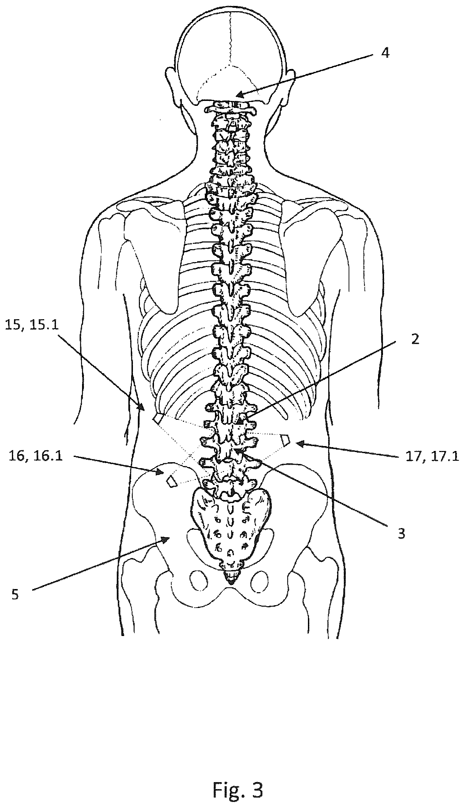

Fig. 3 zeigt eine weitere mögliche Anordnung von Detektionseinrichtungen 15-17 bzw. zugeordneten Detektionsmitteln 15.1-17.1 der erfindungsgemässen Vorrichtung innerhalb des Körpers. Gemäss dieser beispielhaften Ausgestaltung sind wenigstens die Detektionsmittel 15.1-17.1 von bespielhaft drei Detektionseinrichtungen 15-17 seitlich links bzw. rechts an der Wirbelsäule 3 angeordnet und so ausgerichtet, dass sie die Aussenkontur derjenigen Bereiche des zu operierenden Knochenbereichs 2 erfassen, die in distaler Richtung der linken und rechten Körperseite zugewandt sind. Selbstverständlich kann die Anordnung der Detektionseinrichtungen 15-17 gemäss Fig. 3 um weitere Detektionseinrichtungen, die innerhalb oder ausserhalb des Körpers angeordnet werden können, ergänzt werden. Insbesondere kann die spezielle Anordnung der Detektionseinrichtungen gemäss Fig. 3 auch mit jener Anordnung von Detektionseinrichtungen gemäss Fig. 1 und 2 kombiniert werden. Im Idealfall kann mit einer entsprechenden "Rundum-Anordnungen von Detektionseinrichtungen innerhalb und ausserhalb des Körpers sogar ein virtuelles Echtzeit-Rundumbild der Aussenkontur des zu operierenden Knochenbereichs erzeugt werden, wodurch die Navigationsgenauigkeit und das Sicherheitsgefühl für den Operateur erheblich verbessert wird.

## Patentansprüche

1. Vorrichtung (1) für die intraoperative, bildgesteuerte Navigation bei chirurgischen Eingriffen an einem zu operierenden Objekt an der Wirbelsäule (3) und/oder im an die Wirbelsäule (3) angrenzenden Thorax-, Becken- (5) oder Kopfbereich (4), umfassend
mehrere nicht-röntgenbasierte Detektionseinrichtungen (11-17), die jeweils dazu ausgebildet sind, verteilt um das zu operierende Objekt (2) im Bereich der Wirbelsäule (3) und/oder im daran angrenzenden Thorax-, Becken- oder Kopfbereich angeordnet zu werden und intraoperativ in Echtzeit Bild- und Lagedaten zu erfassen, die jeweils Informationen über zumindest die Aussenkontur wenigstens eines Teilbereichs des zu operierenden Objekts (2) sowie über den Abstand und die räumliche Orientierung des Teilbereichs relativ zur jeweiligen Detektionseinrichtung (11-17) umfassen,
eine Positionsbestimmungseinrichtung (20), die dazu ausgebildet ist, in Echtzeit Daten zur jeweiligen Position der Detektionseinrichtungen (11-17) relativ zu einem stationären Bezugssystem (30) zu ermitteln,
eine mit den Detektionseinrichtungen (11-17) und der Positionsbestimmungseinrichtung (20) wirkverbundene Datenverarbeitungseinrichtung (40), die dazu ausgebildet ist, auf Basis der jeweiligen Bild- und Lagedaten der Detektionseinrichtungen (11-17) und der jeweiligen Positionsdaten der Positionsbestimmungseinrichtung (20) ein auf das stationäre Bezugssystem (30) referenziertes, virtuelles Echtzeit-Bild (41) des zu operierenden Objekts (2) zu erzeugen,
eine mit der Datenverarbeitungseinrichtung (40) wirkverbundene Bildanzeigeeinrichtung (50) zur Darstellung des virtuellen Echtzeit-Bildes (41),
wobei wenigstens zwei der mehreren nicht-röntgenbasierten Detektionseinrichtungen (13-17) jeweils eine ultraschallbasierte Detektionseinrichtung oder eine auf Terahertzstrahlung basierte Detektionseinrichtung sind und jeweils wenigstens ein Detektionsmittel (13.1-17.1) aufweisen, das dazu ausgebildet ist, im Inneren des Körpers angeordnet zu werden, um Bilddaten zu erfassen, die Informationen über zumindest die Aussenkontur wenigstens eines dem Körperinneren zugewandten Teilbereichs des zu operierenden Objekts (2) umfassen,
wobei die mehreren nicht-röntgenbasierten Detektionseinrichtungen (11-17) derart verteilt um das zu operierende Objekt (2) angeordnet werden können, dass mittels der
mehreren nicht-röntgenbasierten Detektionseinrichtungen (11-17) intraoperativ in Echtzeit Bild- und Lagedaten für die Aussenkonturen verschiedener Teilbereiche des zu operierenden Objekts erfassbar sind,
wobei die Datenverarbeitungseinrichtung (40) ausgebildet ist, das Echtzeit-Bild (41) des zu operierenden Objekts (2) als ein dreidimensionales Echtzeit-Bild der Aussenkontur des zu operierenden Objekts (2) zu erzeugen, welches aus den Bilddaten für die Aussenkonturen der verschiedenen Teilbereiche des zu operierenden Objekts (2) zusammengesetzt ist.

2. Vorrichtung (1) nach Anspruch 1, wobei die Datenverarbeitungseinrichtung (40) dazu ausgebildet ist, präoperativ gewonnene Bilddaten des zu operierenden Objekts mit den Bilddaten der Detektionseinrichtungen (13-17) zu überlagern, um auf Basis der mit den Bilddaten der Detektionseinrichtungen (13-17) überlagerten, präoperativ gewonnenen Bilddaten das virtuelle, auf das stationäre Bezugssystem referenzierte Echtzeit-Bild des zu operierenden Objekts zu erzeugen.

3. Vorrichtung (1) nach einem der vorhergehenden Ansprüche, wobei das wenigstens eine im Inneren des Körpers anordenbare Detektionsmittel (13.1-17.1) der wenigstens einen Detektionseinrichtung (13-17) dazu ausgebildet ist, über eine präformierte Körperhöhle, insbesondere die Nase, den Mund, den Rachenraum, die Speiseröhre, die Luftröhre, den Magen-Darmtrakt, die Harnblase oder über ein Blutgefäss in den Körper eingebracht zu werden.

4. Vorrichtung (1) nach einem der vorhergehenden Ansprüche, wobei das wenigstens eine im Inneren des Körpers anordenbare Detektionsmittel (13.1-17.1) der wenigstens einen Detektionseinrichtung (13-17) dazu ausgebbildet ist, operativ in ein Gewebe, beispielsweise in paravertebrales Muskelgewebe oder Muskelgewebe des Gesässmuskels, und/oder in Fettgewebe, beispielsweise den Epiduralraum, eingebracht zu werden.

5. Vorrichtung (1) nach einem der vorhergehenden Ansprüche, wobei wenigstens eine der mehreren Detektionseinrichtungen (11, 12) und/oder wenigstens ein Detektionsmittel (11.1, 12.1) wenigstens einer der mehreren Detektionseinrichtungen (11, 12) dazu ausgebildet ist, ausserhalb des Körpers angeordnet zu werden, um Bilddaten zu erfassen, die Informationen über zumindest die Aussenkontur eines dem Körperäusseren zugewandten Teilbereichs des zu operierenden Objekts (2) umfassen.

6. Vorrichtung (1) nach Anspruch 5, wobei die wenigstens eine ausserhalb des Körpers anordenbare Detektionseinrichtung (11, 12) und/oder das wenigstens eine ausserhalb des Körpers anordenbare Detektionsmittel (11.1, 12.1) eine ultraschallbasierte Detektionseinrichtung oder ein optischer Konturscanner oder eine auf Terahertzstrahlung basierte Detektionseinrichtung ist.

7. Vorrichtung (1) nach einem der vorhergehenden Ansprüche, wobei die Vorrichtung (1) wenigstens ein Operationswerkzeug (60) umfasst, wobei die Positionsbestimmungseinrichtung (20) ferner dazu ausgebildet ist, in Echtzeit Daten zur Position des wenigstens einen Operationswerkzeuges (60) relativ zum stationären Bezugsystem (30) zu ermitteln, und wobei die Datenverarbeitungseinheit (40) ferner zum Einblenden eines das Operationswerkzeug (60) und dessen Lage repräsentierenden Indikators in das virtuelle Echtzeit-Bild (41) ausgebildet ist.

8. Vorrichtung nach einem der vorhergehenden Ansprüche, insbesondere nach Anspruch 7, wobei wenigstens eine der mehreren Detektionseinrichtungen (11-17) und/oder wenigstens ein Detektionsmittel (11.1-17.1) wenigstens einer der mehreren Detektionseinrichtungen (11-17) dazu ausgebildet ist, an einem Operationswerkzeug (60) angeordnet zu werden, insbesondere dass wenigstens eine der mehreren Detektionseinrichtungen (11-17) und/oder wenigstens ein Detektionsmittel (11.1-17.1) wenigstens einer der mehreren Detektionseinrichtungen (11-17) an dem Operationswerkzeug (60) angeordnet ist.

9. Vorrichtung (1) nach einem der Ansprüche 1 bis 8, wobei die Datenverarbeitungseinrichtung (40) dazu ausgebildet ist, bei einem temporären Ausfall der Bilddaten-, der Lagedaten und/oder der Positionsdatenermittlung wenigstens einer der Detektionseinrichtungen (11-17) den von dieser Detektionseinrichtung (11-17) erfassten Teilbereich des zu operierenden Objekts (2) in dem virtuellen Echtzeit-Bild (41) auf Basis der zu einem früheren Zeitpunkt von dieser Detektionseinrichtung (11-17) bei funktionierender Lagedaten- und Positionsdatenermittlung erfassten Bilddaten zu erzeugen, wobei die zu einem früheren Zeitpunkt erfassten Bilddaten des entsprechenden Teilbereichs in Echtzeit an die aktuelle Lage der von den übrigen Detektionseinrichtungen (11-17) erfassten Teilbereiche anpasst werden.

10. Vorrichtung (1) nach einem der Ansprüche 1 bis 8, wobei die Datenverarbeitungseinrichtung (40) dazu ausgebildet ist, bei einem temporären Ausfall der Lagedaten- und/oder Positionsdatenermittlung einer Detektionseinrichtung (11-17) den von dieser Detektionseinrichtung (11-17) erfassten Teilbereich des zu operierenden Objekts (2) in dem virtuellen Echtzeit-Bild (41) auf Basis der aktuell erfassten Bilddaten zu erzeugen, wobei die aktuell erfassten Bilddaten des entsprechenden Teilbereichs in Echtzeit an die aktuelle Lage der von den übrigen Detektionseinrichtungen (11-17) erfassten Teilbereiche anpasst werden.

11. Vorrichtung (1) nach einem der Ansprüche 1 bis 10, wobei die Vorrichtung (1) ferner wenigstens eine röntgenbasierte Detektionseinrichtung aufweist, die dazu ausgebildet ist, intraoperativ in Echtzeit Bilddaten zu erfassen, die Informationen über die Aussenkontur und innere Struktur wenigstens eines Teilbereichs des zu operierenden Objekts (2) umfassen, wobei die Datenverarbeitungseinrichtung (40) mit der röntgenbasierten Detektionseinrichtung wirkverbunden ist und dazu ausgebildet ist, die Bilddaten der röntgenbasierten Detektionseinrichtung mit den Bilddaten der nicht-röntgenbasierten Detektionseinrichtungen (13-17) zu überlagern, um auf Basis der miteinander überlagerten Bilddaten das virtuelle auf das stationäre Bezugssystem referenzierte Echtzeit-Bild des zu operierenden Objekts zu erzeugen, das Informationen über die Aussenkontur und innere Struktur des zu operierenden Objekts umfasst.

12. Vorrichtung (1) nach einem der Ansprüche 1 bis 10, wobei die Vorrichtung (1) ferner wenigstens eine röntgenbasierte Detektionseinrichtung aufweist, die dazu ausgebildet ist, intraoperativ in Echtzeit Bild- und Lagedaten zu erfassen, die Informationen über die Aussenkontur und innere Struktur wenigstens eines Teilbereichs des zu operierenden Objekts (2) sowie über die Lage des Teilbereichs relativ zur röntgenbasierten Detektionseinrichtung umfassen, wobei die Positionsbestimmungseinrichtung (20) ferner dazu ausgebildet ist, in Echtzeit Daten zur Position der röntgenbasierten Detektionseinrichtung relativ zum stationären Bezugssystem (30) zu ermitteln, und wobei die Datenverarbeitungseinrichtung (40) ferner mit der röntgenbasierten Detektionseinrichtung wirkverbunden ist und dazu ausgebildet ist, auf Basis der jeweiligen Bild- und Lagedaten der nicht-röntgenbasierten Detektionseinrichtungen (11-17), der Bild- und Lagedaten der röntgenbasierten Detektionseinrichtung und der jeweiligen Positionsdaten der Positionsbestimmungseinrichtung (20) ein auf das stationäre Bezugssystem (30) referenziertes, virtuelles, vorzugsweise dreidimensionales Echtzeit-Bild (41) des zu operierenden Objekts (2) zu erzeugen, das Informationen über die Aussenkontur und innere Struktur des zu operierenden Objekts umfasst.

13. Vorrichtung nach einem der vorhergehenden Ansprüche, wobei die Detektionseinrichtungen (13-17) dazu ausgebildet sind, miteinander zu kommunizieren, insbesondere Informationen über ihre jeweilige Position und/oder Lage, vorzugsweise relativ zum stationären Bezugssystem (30), auszutauschen.

14. Vorrichtung nach einem der vorhergehenden Ansprüche, wobei wenigstens eine der mehreren Detektionseinrichtungen (13-17), insbesondere wenigstens ein Detektionsmittel (13.1-17.1) wenigstens einer der Detektionseinrichtungen (13-17), dazu ausgebildet ist, die Position und/oder Lage wenigstens einer anderen Detektionseinrichtung (13-17) bzw. die Position und/oder Lage des jeweiligen Detektionsmittels (13.1-17.1) der wenigstens einen anderen Detektionseinrichtung (13-17) relativ zu sich, und vorzugsweise dadurch auch über die Positionsbestimmungseinrichtung (20) relativ zum stationären Bezugssystem (30), zu ermitteln.

## Claims

1. A device (1) for intraoperative, image-controlled navigation during surgical procedures on an object to be operated on on the spinal column (3) and/or in the thorax, pelvis (5) or head (4) region adjacent to the spinal column (3), comprising:
multiple detection devices (11 - 17) that are not x-ray based, which are each configured to be arranged such that they are distributed about the object (2) to be operated on, in the region of the spinal column (3) and/or in the thorax, pelvis or head region adjacent thereto, and to intraoperatively capture image- and location data in real time, respectively comprising information relating to at least the outer contour of at least one subregion of the object (2) to be operated on, and relating to the distance and the spatial orientation of the subregion relative to the respective detection device (11 - 17),
a position determining device (20), which is configured to determine in real time data relating to the respective position of the detection devices (11 - 17) relative to a stationary reference system (30),
a data processing device (40) in operative connection with the detection devices (11 - 17) and with the position determining device (20), which data processing device is configured to generate, on the basis of the respective image- and location data of the detection devices (11 - 17) and of the respective position data of the position determining device (20), a virtual real time image (41) of the object (2) to be operated on, referenced to the stationary reference system (30), an image display device (50) in operative connection with the data processing device (40), for displaying the virtual real time image (41),
wherein at least two of the multiple detection devices (13-17) that are not x-ray based are respectively an ultrasound-based detection device or a detection device based on terahertz radiation and respectively comprise at least one detection means (13.1-17.1) which is configured to be arranged inside the body, in order to capture image data which comprise information relating to at least the outer contour of at least one subregion of the object (2) to be operated on, facing the inside of the body,
wherein the multiple detection devices (11-17) that are not x-ray based can be arranged distributed about the object (2) which is to be operated on, such that by means of the multiple detection devices (11-17) that are not x-ray based intraoperatively in real time image- and location data for the outer contours of various subregions of the object to be operated on are able to be captured,
wherein the data processing device (40) is configured to generate the real time image (41) of the object (2) to be operated on as a three-dimensional real time image of the outer contour of the object (2) to be operated on, which is composed of the image data for the outer contours of the various subregions of the object (2) to be operated on.

2. The device (1) according to Claim 1, wherein the data processing device (40) is configured to superimpose preoperatively obtained image data of the object to be operated on with the image data of the detection devices (13 - 17), in order to generate the virtual real time image of the object to be operated on, referenced to the stationary reference system, on the basis of the preoperatively obtained image data, superimposed with the image data of the detection devices (13 - 17).

3. The device (1) according to one of the preceding claims, wherein the at least one detection means (13.1 - 17.1), able to be arranged inside the body, of the at least one detection device (13 - 17) is configured to be introduced into the body via a preformed body cavity, in particular the nose, the mouth, the pharyngeal cavity, the oesophagus, the trachea, the gastrointestinal tract, the urinary bladder, or via a blood vessel.

4. The device (1) according to one of the preceding claims, wherein the at least one detection means (13.1 - 17.1), able to be arranged inside the body, of the at least one detection device (13 - 17) is configured to be introduced operatively into a tissue, for example into paravertebral muscular tissue or muscular tissue of the gluteal muscle, and/or into adipose tissue, for example into the epidural space.

5. The device (1) according to one of the preceding claims,
wherein at least one of the multiple detection devices (11, 12) and/or at least one detection means (11.1, 12.1) of at least one of the multiple detection devices (11, 12) is configured to be arranged outside the body, in order to capture image data which comprise information relating to at least the outer contour of a subregion of the object (2) to be operated on, facing the outside of the body.

6. The device (1) according to Claim 5, wherein the at least one detection device (11, 12), able to be arranged outside the body, and/or the at least one detection means (11.1, 12.1), able to be arranged outside the body, is an ultrasound-based detection device or an optical contour scanner or a detection device based on terahertz radiation.

7. The device (1) according to one of the preceding claims, wherein the device (1) comprises at least one operating instrument (60), wherein the position determining device (20) is, in addition, configured to determine in real time data relating to the position of the at least one operating instrument (60) relative to the stationary reference system (30), and wherein the data processing unit (40) is, in addition, configured to superimpose an indicator, representing the operating instrument (60) and its location, into the virtual real time image (41).

8. The device according one of the preceding claims, in particular according to Claim 7, wherein at least one of the multiple detection devices (11 - 17) and/or at least one detection means (11.1 - 17.1) of at least one of the multiple detection devices (11 - 17) is configured to be arranged on an operating instrument (60), in particular that at least one of the multiple detection devices (11 - 17) and/or at least one detection means (11.1 - 17.1) of at least one of the multiple detection devices (11 - 17) is arranged on the operating instrument (60).

9. The device (1) according to one of Claims 1 to 8, wherein the data processing device (40) is configured, in the case of a temporary failure of the determining of image data, location data and/or position data of at least one of the detection devices (11 - 17), to generate the subregion of the object (2) to be operated on, captured by this detection device (11 - 17), in the virtual real time image (41) on the basis of the image data captured at an earlier point in time by this detection device (11 - 17) with functioning determining of location data and position data, wherein the image data of the corresponding subregion captured at an earlier point in time are adapted in real time to the current location of the subregions captured by the remaining detection devices (11 - 17).

10. The device (1) according to one of Claims 1 to 8, wherein the data processing device (40) is configured, in the case of a temporary failure of the determining of location data and/or position data of a detection device (11 - 17), to generate the subregion of the object (2) to be operated on, captured by this detection device (11 - 17), in the virtual real time image (41) on the basis of the currently captured image data, wherein the currently captured image data of the corresponding subregion are adapted in real time to the current location of the other subregions captured by the remaining detection devices (11 - 17).

11. The device (1) according to one of Claims 1 to 10, wherein the device (1) comprises, in addition, at least one x-ray based detection device, which is configured to intraoperatively capture image data in real time which comprise information relating to the outer contour and inner structure of at least one subregion of the object (2) to be operated on, wherein the data processing device (40) is in operative connection with the x-ray based detection device and is configured to superimpose the image data of the x-ray based detection device with the image data of the detection devices (13 - 17) that are not x-ray based, in order, on the basis of the image data which are superimposed with one another, to generate the virtual real time image of the object to be operated on, referenced to the stationary reference system, which comprises information relating to the outer contour and inner structure of the object to be operated on.

12. The device (1) according to one of Claims 1 to 10, wherein the device (1) comprises in addition at least one x-ray based detection device, which is configured to intraoperatively capture in real time image- and location data which comprise information relating to the outer contour and inner structure of at least one subregion of the object (2) to be operated on and relating to the location of the subregion relative to the x-ray based detection device, wherein the position determining device (20) is in addition configured to determine in real time data relating to the position of the x-ray based detection device relative to the stationary reference system (30), and wherein the data processing device (40) is, in addition, in operative connection with the x-ray based detection device and is configured, on the basis of the respective image- and location data of the non-x-ray based detection devices (11 - 17), of the image- and location data of the x-ray based detection device and of the respective position data of the position determining device (20), to generate a virtual, preferably three-dimensional real time image (41) of the object (2) to be operated on, referenced to the stationary reference system (30), which image comprises information relating to the outer contour and inner structure of the object to be operated on.

13. The device according to one of the preceding claims, wherein the detection devices (13 - 17) are configured to communicate with one another, in particular to exchange information relating to their respective position and/or location, preferably relative to the stationary reference system (30).

14. The device according to one of the preceding claims, wherein at least one of the multiple detection devices (13 - 17), in particular at least one detection means (13.1 - 17.1) of at least one of the detection devices (13 - 17), is configured to determine the position and/or location of at least one other detection device (13 - 17) or respectively the position and/or location of the respective detection means (13.1 - 17.1) of the at least one other detection device (13 - 17) relative to itself, and preferably thereby via the position determining device (20) also relative to the stationary reference system (30).

## Revendications

1. Dispositif (1) pour la navigation intra-opératoire, pilotée par images dans les interventions chirurgicales sur un objet à opérer sur la colonne vertébrale (3) et/ou au niveau du thorax, du bassin (5) ou de la tête (4) adjacent à la colonne vertébrale (3), comprenant
une pluralité de dispositifs de détection non basés sur des radiographies (11-17) qui sont respectivement conçus pour être disposés répartis autour de l'objet à opérer (2) au niveau de la colonne vertébrale (3) et/ou au niveau du thorax, du bassin ou de la tête au niveau de la colonne vertébrale (3) et pour collecter des données d'images et de situation en temps réel en intra-opératoire, qui comprennent respectivement des informations sur au moins le contour extérieur d'au moins une région partielle de l'objet à opérer (2) ainsi que sur la distance et l'orientation spatiale de la région partielle par rapport au dispositif de détection (11-17) respectif,
un dispositif de détermination de la position (20) qui est conçu pour déterminer en temps réel des données sur la position respective des dispositifs de détection (11-17) par rapport à un système de référence stationnaire (30),
un dispositif de traitement des données (40) relié en coopération aux dispositifs de détection (11-17) et au dispositif de détermination de la position (20) qui est conçu pour produire une image virtuelle en temps réel (41) référencée sur le système de référence (30) stationnaire de l'objet à opérer (2), en se basant sur les données d'images et de situation respectives des dispositifs de détection (11-17) et les données de position respectives du dispositif de détermination de la position (20),
un dispositif d'affichage d'images (50) relié en coopération au dispositif de traitement des données (40) pour représenter l'image virtuelle en temps réel (41),
dans lequel au moins deux des dispositifs de détection non basés sur des radiographies (13-17) sont respectivement un dispositif de détection à base d'ultrasons ou un dispositif de détection à base de rayonnement térahertz et présentent respectivement au moins un moyen de détection (13.1-17.1) qui est conçu pour être disposé à l'intérieur du corps pour collecter des données d'images qui comportent des informations sur au moins le contour extérieur d'au moins une région partielle de l'objet à opérer (2) tournée vers l'intérieur du corps,
dans lequel les dispositifs de détection non basés sur des radiographies (11-17) peuvent être ainsi disposés répartis autour de l'objet à opérer (2) qu'au moyen de la pluralité de dispositifs de détection non basés sur des radiographies (11-17), des données d'images et de situation pour les contours extérieurs de différentes régions partielles de l'objet à opérer peuvent être collectées en temps réel de manière intra-opératoire,
dans lequel le dispositif de traitement des données (40) est conçu pour produire l'image en temps réel (41) de l'objet à opérer (2) en tant qu'une image en temps réel tridimensionnelle du contour extérieur de l'objet à opérer (2), laquelle est composée des données d'images pour les contours extérieurs des différentes régions partielles de l'objet à opérer (2).

2. Dispositif (1) selon la revendication 1, dans lequel le dispositif de traitement des données (40) est conçu pour superposer des données d'images acquises en préopératoire de l'objet à opérer avec les données d'images des dispositifs de détection (13-17) pour produire l'image virtuelle en temps réel référencée sur le système de référence stationnaire de l'objet à opérer, en se basant sur les données d'images acquises en préopératoire superposées avec les données d'images des dispositifs de détection (13-17) .

3. Dispositif (1) selon l'une des revendications précédentes, dans lequel l'au moins un moyen de détection (13.1-17.1) pouvant être agencé à l'intérieur du corps de l'au moins un dispositif de détection (13-17) est conçu pour être introduit dans le corps par une cavité corporelle préformée, en particulier le nez, la bouche, le pharynx, l'œsophage, la trachée, le système gastro-intestinal, la vessie ou par un vaisseau sanguin.

4. Dispositif (1) selon l'une des revendications précédentes, dans lequel l'au moins un moyen de détection (13.1-17.1) pouvant être agencé à l'intérieur du corps de l'au moins un dispositif de détection (13-17) est conçu pour être introduit de manière opératoire dans un tissu, par exemple dans un tissu musculaire para-vertébral ou un tissu musculaire du muscle fessier, et/ou dans les tissus adipeux, par exemple dans l'espace épidural.

5. Dispositif (1) selon l'une des revendications précédentes, dans lequel au moins un de la pluralité de moyens de détection (11, 12) et/ou au moins un moyen de détection (11.1, 12.1) d'au moins un de la pluralité de moyens de détection (11, 12) est conçu pour être disposé en-dehors du corps, pour collecter des données d'images qui comprennent des informations sur au moins le contour extérieur d'une région partielle de l'objet à opérer (2) tournée vers l'extérieur du corps.

6. Dispositif (1) selon la revendication 5, dans lequel l'au moins un dispositif de détection (11, 12) pouvant être agencé à l'extérieur du corps et/ou l'au moins un moyen de détection (11.1, 12.1) pouvant être agencé à l'extérieur du corps, est un dispositif de détection à base d'ultrasons ou un scanner de contour optique ou un dispositif de détection à base de rayonnement térahertz.

7. Dispositif (1) selon l'une des revendications précédentes, dans lequel le dispositif (1) comprend au moins un outil opérationnel (60), dans lequel le dispositif de détermination de la position (20) est en outre conçu pour déterminer en temps réel des données sur la position de l'au moins un outil opérationnel (60) par rapport au système de référence stationnaire (30), et dans lequel le dispositif de traitement des données (40) est conçu en outre pour afficher un indicateur représentant l'outil opérationnel (60) et sa situation dans l'image virtuelle en temps réel (41).

8. Dispositif selon l'une des revendications précédentes, en particulier la revendication 7, dans lequel au moins un de la pluralité de dispositifs de détection (11-17) et/ou au moins un moyen de détection (11.1, 17.1) d'au moins un de la pluralité de dispositifs de détection (11-17) est conçu pour être disposé sur un outil opérationnel (60), en particulier en ce qu'au moins un de la pluralité de dispositifs de détection (11-17) et/ou au moins un moyen de détection (11.1, 17.1) d'au moins un de la pluralité de dispositifs de détection (11-17) est disposé sur l'outil opérationnel (60).

9. Dispositif (1) selon l'une des revendications 1 à 8, dans lequel le dispositif de traitement des données (40) est conçu, en cas de défaillance temporaire de la collecte des données d'images, des données de situation et/ou des données de position d'au moins un des dispositifs de détection (11-17), pour produire la région partielle de l'objet à opérer (2) détectée par ce dispositif de détection (11-17) dans l'image virtuelle en temps réel (41) en se basant sur les données d'images collectées à un moment antérieur par ce dispositif de détection (11-17) lorsque la collecte de données de situation et de données de position fonctionnait, dans lequel les données d'images collectées à un moment antérieur de la région partielle correspondante sont adaptées en temps réel à la situation actuelle des régions partielles collectées par les dispositifs de détection (11-17) restants.

10. Dispositif (1) selon l'une des revendications 1 à 8, dans lequel le dispositif de traitement des données (40) est conçu, en cas de panne temporaire de la collecte des données de situation et/ou des données de position d'un des dispositifs de détection (11-17), pour produire la région partielle de l'objet à opérer (2) détectée par ce dispositif de détection (11-17) dans l'image virtuelle en temps réel (41) en se basant sur les données d'images collectées actuellement, dans lequel les données d'images collectées actuellement de la région partielle correspondante sont adaptées en temps réel à la situation actuelle des régions partielles collectées par les dispositifs de détection (11-17) restants.

11. Dispositif (1) selon l'une des revendications 1 à 10, dans lequel le dispositif (1) présente en outre au moins un dispositif de détection basé sur des radiographies qui est conçu pour collecter des données d'images en temps réel en intra-opératoire, qui comportent des informations sur le contour extérieur et la structure intérieure d'au moins une région partielle de l'objet à opérer (2), dans lequel le dispositif de traitement des données (40) est relié en coopération au dispositif de détection basé sur des radiographies et est conçu pour superposer les données d'images du dispositif de détection basé sur des radiographies aux données d'images des dispositifs de détection non basés sur des radiographies (13-17), pour produire l'image virtuelle en temps réel de l'objet à opérer référencée sur le système de référence, en se basant sur les données d'images superposées entre elles, laquelle comprend des informations sur le contour extérieur et la structure intérieure de l'objet à opérer.

12. Dispositif (1) selon l'une des revendications 1 à 10, dans lequel le dispositif (1) présente en outre au moins un dispositif de détection basé sur des radiographies qui est conçu pour collecter des données d'images en temps réel en intra-opératoire, qui comportent des informations sur le contour extérieur et la structure intérieure d'au moins une région partielle de l'objet à opérer (2) ainsi que sur la situation de la région partielle par rapport au dispositif de détection basé sur des radiographies, dans lequel le dispositif de détermination de la position (20) est conçu en outre pour déterminer en temps réel des données sur la position de dispositif de détection basé sur des radiographies par rapport au système de référence stationnaire (30), et dans lequel le dispositif de traitement des données (40) est en outre relié en coopération au dispositif de détection basé sur des radiographies et conçu pour, en se basant sur les données d'images et de situation respectives des dispositifs de détection non basés sur des radiographies (11-17), les données d'images et de situation du dispositif de détection basé sur des radiographies et les données de position respectives du dispositif de détermination de la position (20), produire une image (41) virtuelle en temps réel, de préférence tridimensionnelle, de l'objet à opérer (2) référencée sur le système de référence (30) stationnaire, laquelle comprend des informations sur le contour extérieur et la structure intérieure de l'objet à opérer.

13. Dispositif (1) selon l'une des revendications précédentes, dans lequel les dispositifs de détection (13-17) sont conçus pour communiquer entre eux, en particulier pour échanger des informations sur leur position et/ou situation respective, de préférence par rapport au système de référence stationnaire (30).

14. Dispositif (1) selon l'une des revendications précédentes, dans lequel au moins un de la pluralité de dispositifs de détection (13-17), en particulier au moins un moyen de détection (13.1-17.1) d'au moins un des dispositifs de détection (13-17) est conçu pour déterminer la position et/ou la situation d'au moins un autre dispositif de détection (13-17), respectivement la position et/ou la situation du moyen de détection (13.1-17.1) respectif de l'au moins un autre dispositif de détection (13-17) par rapport à soi, et de préférence également par le dispositif de détermination de la position (20) par rapport au système de référence stationnaire (30).
